# EUROPEAN PATENT APPLICATION

(11) **EP 4 834 364 A1**
(43) Date of publication of application: **04.11.2026**
(21) Application number: 24911416.6
(22) Date of filing: 27.12.2024
(51) Int. Cl.: A61K 39/395, A61K 47/26, A61K 47/34, A61K 47/18, A61K 9/19, A61P 35/00

(54) **STABLE PHARMACEUTICAL COMPOSITION OF ANTI-PD-1 ANTIBODY**

(30) Priority: 28.12.2023 CN 202311848745; 20.12.2024 CN 202411895668
(71) Applicant: QILU PHARMACEUTICAL CO., LTD., Jinan, Shandong 250100 (CN)
(72) Inventor: YANG, Fusheng, Jinan, Shandong 250100 (CN); ZHANG, Le, Jinan, Shandong 250100 (CN); AN, Zhenming, Jinan, Shandong 250100 (CN); SUN, Lixia, Jinan, Shandong 250100 (CN); LIU, Jun, Jinan, Shandong 250100 (CN); ZHOU, Xin, Jinan, Shandong 250100 (CN); YANG, Chen, Jinan, Shandong 250100 (CN); ZHOU, Bingbing, Jinan, Shandong 250100 (CN); XIE, Wei, Jinan, Shandong 250100 (CN); LIU, Yapu, Jinan, Shandong 250100 (CN); CHEN, Junliang, Jinan, Shandong 250100 (CN)
(74) Representative: Studio Torta S.p.A.
(86) International application number: PCT/CN2024/143014
(87) International publication number: WO 2025/140495

(57) **Abstract**

Provided is a stable pharmaceutical composition comprising an anti-PD-1 antibody. More particularly, the present invention relates to a pharmaceutical composition of pembrolizumab. The stability of the composition is ensured while the viscosity of a formulation is reduced, effectively solving the problems of high-concentration antibody formulations in drug manufacturing, storage and transportation, and drug delivery.

## Description

The present application claims priority to the Chinese Patent Application No. 202311848745.9 entitled "STABLE PHARMACEUTICAL COMPOSITION OF ANTI-PD-1 ANTIBODY" and filed with China National Intellectual Property Administration on December 28, 2023 and the Chinese Patent Application No. 202411895668.7 entitled "STABLE PHARMACEUTICAL COMPOSITION OF ANTI-PD-1 ANTIBODY" and filed with China National Intellectual Property Administration on December 20, 2024, which are incorporated herein by reference in their entirety.

### TECHNICAL FIELD

The present disclosure relates to a stable pharmaceutical composition of an anti-PD-1 antibody, and more particularly, the present disclosure relates to a pharmaceutical composition of pembrolizumab and therapeutic use thereof.

### BACKGROUND

Pembrolizumab is an anti-PD-1 humanized monoclonal antibody and was approved by the FDA for marketing in 2014. The specification of the formulation is 100 mg:4 mL, and the administration route is intravenous infusion. In recent years, the subcutaneous administration route has attracted attention in the field of biomacromolecules for its advantages such as convenience and relatively good patient compliance. In order to improve the convenience of administration for patients, the use of high-concentration protein solutions (i.e., >80 mg/mL) in prefilled syringes has become a trend. At such concentrations, many protein solutions become highly viscous, posing significant challenges for handling and delivery. For subcutaneous injections, particularly when prefilled syringes are used, formulation formulas having a solution viscosity of less than 15 centipoise (cP) are preferred. Otherwise, the injection force (also referred to as glide force) may increase, which is a challenge for high-concentration solutions. Meanwhile, in ultrafiltration/diafiltration systems, high viscosity may lead to high membrane back pressure and reduced flow rates. In prefilled syringes, increased viscosity may result in difficulties during administration. For example, injection of highly viscous solutions may increase leakage at the injection site and require the use of needles with larger diameters, thereby increasing patient pain. For lyophilized high-concentration formulations, high viscosity may also cause the problem of prolonged reconstitution time. Therefore, controlling solution viscosity is an urgent issue in the development of high-concentration protein formulations. In biologic formulation formulas, sucrose or trehalose is commonly used as a stabilizer. However, relatively high concentrations of sucrose or trehalose usually lead to relatively high formulation viscosity. If their amounts are reduced during the development of high-concentration formulations in order to control product viscosity, the reduction in the amount of the stabilizer may potentially affect product stability. Therefore, the high viscosity problem associated with high-concentration biologic formulations poses numerous challenges to drug manufacturing, storage and transportation, and drug delivery.

At present, studies have shown that amino acid excipients may replace sucrose to achieve the effect of a stabilizer. MERCK SHARP & DOHME disclosed in patent WO2019050780A1 a novel PEGylated amino acid capable of reducing the viscosity of high-concentration biologic formulations. In another patent, US11633476B2, the specification describes a high-concentration formulation formula in which a single amino acid is used as a stabilizer. However, the results generally fail to simultaneously achieve a balance among stability, viscosity properties, insoluble microparticles, and other aspects of the formulated product. Consequently, in its claims, only the high-concentration formulation formula using sucrose as the stabilizer was ultimately allowed. Therefore, there is an urgent need to further optimize the formula of high-concentration formulations, particularly to optimize the components of the stabilizer, so as to achieve control of the viscosity of high-concentration formulations while ensuring product stability, thereby meeting the requirements of industrial production.

### SUMMARY

In order to achieve a better viscosity-reducing effect and a more stable formulation composition, the present disclosure obtained, through formula screening, a pembrolizumab antibody formulation formula containing an amino acid system. By using a combination of amino acids or a combination of an amino acid and sucrose as a stabilizer and optimizing a ratio to obtain an optimal composition ratio, the formulations containing the amino acid system of the present disclosure exhibit superior SEC purity and IEC purity as compared with conventional formulations, and improve product stability while effectively reducing formulation viscosity, thereby facilitating drug delivery.

The present disclosure provides a pharmaceutical composition of an anti-PD-1 antibody, which comprises
a) an anti-PD-1 antibody at about 80-200 mg/mL;
b) a surfactant, wherein the surfactant is selected from polysorbate 80, polysorbate 20, and poloxamer 188;
c) a stabilizer, wherein the stabilizer is a combination of arginine and proline, or a combination of arginine and sucrose;
d) an antioxidant, wherein the antioxidant is methionine;
e) a diffusing agent, wherein the diffusing agent is hyaluronidase,
wherein pH of the pharmaceutical composition is about 4.0-6.0, a content of the arginine is about 0.1-50 mg/mL, a content of the proline is about 0.1-50 mg/mL, and a content of the sucrose is about 0.1-50 mg/mL.

In some embodiments, in the pharmaceutical composition of the present disclosure, the anti-PD-1 antibody is in a high-concentration state, wherein a concentration of the anti-PD-1 antibody is about 80-200 mg/mL, for example, the pharmaceutical composition comprises the anti-PD-1 antibody at about 80 mg/mL, 85 mg/mL, 90 mg/mL, 95 mg/mL, 100 mg/mL, 105 mg/mL, 110 mg/mL, 115 mg/mL, 120 mg/mL, 125 mg/mL, 130 mg/mL, 135 mg/mL, 140 mg/mL, 145 mg/mL, 150 mg/mL, 155 mg/mL, 160 mg/mL, 165 mg/mL, 170 mg/mL, 175 mg/mL, 180 mg/mL, 185 mg/mL, 190 mg/mL, 195 mg/mL, or 200 mg/mL.

In some embodiments, in the pharmaceutical composition of the present disclosure, the stabilizer is a combination of arginine and proline, wherein the contents of the arginine and the proline are each about 0.1-50 mg/mL, for example, the content of the arginine is about 50 mg/mL, 45 mg/mL, 40 mg/mL, 35 mg/mL, 30 mg/mL, 20 mg/mL, 15 mg/mL, 10 mg/mL, 5 mg/mL, 1 mg/mL, or 0.1 mg/mL, preferably about 15 mg/mL, and the content of the proline is about 50 mg/mL, 45 mg/mL, 40 mg/mL, 35 mg/mL, 30 mg/mL, 20 mg/mL, 15 mg/mL, 10 mg/mL, 5 mg/mL, 1 mg/mL, or 0.1 mg/mL, preferably about 15 mg/mL. In some embodiments, a weight ratio of the arginine to the proline is about 1:2 to 2:1, preferably about 1:2, 1:1, or 2:1.

In some embodiments, in the pharmaceutical composition of the present disclosure, the stabilizer is a combination of arginine and sucrose, wherein the contents of the arginine and the sucrose are each about 0.1-50 mg/mL, for example, the content of the arginine is about 50 mg/mL, 45 mg/mL, 40 mg/mL, 35 mg/mL, 30 mg/mL, 20 mg/mL, 15 mg/mL, 10 mg/mL, 5 mg/mL, 1 mg/mL, or 0.1 mg/mL, preferably about 20 mg/mL, and the content of the sucrose is about 50 mg/mL, 45 mg/mL, 40 mg/mL, 35 mg/mL, 30 mg/mL, 20 mg/mL, 15 mg/mL, 10 mg/mL, 5 mg/mL, 1 mg/mL, or 0.1 mg/mL, preferably about 20 mg/mL. In some embodiments, a weight ratio of the arginine to the sucrose is about 1:2 to 2:1, preferably about 1:2, 1:1, or 2:1.

In some embodiments, in the pharmaceutical composition of the present disclosure, a concentration of methionine is about 0.1-50 mM, for example, the concentration of methionine may be about 0.1 mM, 1 mM, 5 mM, 10 mM, 15 mM, 20 mM, 25 mM, 30 mM, 35 mM, 40 mM, 45 mM, or 50 mM, and preferably, the concentration of methionine is about 15 mM. Optionally, nitrogen is introduced into the pharmaceutical composition.

In some embodiments, in the pharmaceutical composition of the present disclosure, the surfactant is selected from polysorbate 80, polysorbate 20, and poloxamer 188, wherein a concentration of the surfactant is about 0.01-1 mg/mL, for example, the concentration of the surfactant may be about 0.01 mg/mL, 0.05 mg/mL, 0.1 mg/mL, 0.2 mg/mL, 0.3 mg/mL, 0.4 mg/mL, 0.5 mg/mL, 0.6 mg/mL, 0.7 mg/mL, 0.8 mg/mL, 0.9 mg/mL, or 1 mg/mL. A preferred surfactant is polysorbate 80, and a concentration of the preferred surfactant is preferably about 0.4 mg/mL.

In some embodiments, in the pharmaceutical composition of the present disclosure, a concentration of hyaluronidase is about 1-10000 IU/mL, preferably about 1-4000 IU/mL, and more preferably about 1-2000 IU/mL.

In some embodiments, the pharmaceutical composition of the present disclosure further comprises a buffer agent.

In some embodiments, in the pharmaceutical composition of the present disclosure, the buffer agent is histidine-hydrochloric acid, wherein a concentration of the buffer agent is about 0.1-30 mM, for example, the concentration of the buffer agent may be about 0.1 mM, 1 mM, 5 mM, 10 mM, 15 mM, 20 mM, 25 mM, or 30 mM, and preferably, the concentration of the buffer agent is about 10 mM.

In some embodiments, the anti-PD-1 antibody of the present disclosure is pembrolizumab, the heavy chain sequence of the anti-PD-1 antibody is shown in SEQ ID NO: 1, and the light chain sequence is shown in SEQ ID NO: 2.

In some preferred embodiments, the present disclosure provides a pharmaceutical composition comprising about 80-200 mg/mL pembrolizumab, about 0.1-30 mM histidine-hydrochloric acid, about 0.01-1 mg/mL polysorbate 80, about 0.1-50 mM methionine, about 0.1-50 mg/mL arginine, about 0.1-50 mg/mL proline, and about 1-10000 IU/mL hyaluronidase, wherein pH of the pharmaceutical composition is about 4.0-6.0, for example, the pH of the pharmaceutical composition may be about 4.0, 4.5, 5, 5.5, or 6.0, and optionally, nitrogen is introduced as required.

Further preferably, the present disclosure provides a pharmaceutical composition of an anti-PD-1 antibody, comprising about 160-180 mg/mL pembrolizumab, about 5-20 mM histidine-hydrochloric acid, about 0.1-0.6 mg/mL polysorbate 80, about 5-25 mM methionine, about 5-20 mg/mL arginine, about 5-20 mg/mL proline, and about 1-4000 IU/mL hyaluronidase, wherein pH of the pharmaceutical composition is about 5.0-6.0, for example, the pH of the pharmaceutical composition may be about 5.0, 5.2, 5.5, 5.7, or 6.0, and optionally, nitrogen is introduced as required.

In some preferred embodiments, the present disclosure provides a pharmaceutical composition comprising about 80-200 mg/mL pembrolizumab, about 0.1-30 mM histidine-hydrochloric acid, about 0.01-1 mg/mL polysorbate 80, about 0.1-50 mM methionine, about 0.1-50 mg/mL arginine, about 0.1-50 mg/mL sucrose, and about 1-10000 IU/mL hyaluronidase, wherein pH of the pharmaceutical composition is about 4.0-6.0, for example, the pH of the pharmaceutical composition may be about 4.0, 4.5, 5, 5.5, or 6.0, and optionally, nitrogen is introduced as required.

Further preferably, the present disclosure provides a pharmaceutical composition of an anti-PD-1 antibody, comprising about 160-180 mg/mL pembrolizumab, about 5-20 mM histidine-hydrochloric acid, about 0.1-0.6 mg/mL polysorbate 80, about 5-25 mM methionine, about 5-25 mg/mL arginine, about 5-25 mg/mL sucrose, and about 1-4000 IU/mL hyaluronidase, wherein pH of the pharmaceutical composition is about 5.0-6.0, for example, the pH of the pharmaceutical composition may be about 5.0, 5.2, 5.5, 5.7, or 6.0, and optionally, nitrogen is introduced as required.

In some preferred embodiments, the present disclosure provides a pharmaceutical composition of an anti-PD-1 antibody, comprising about 160-180 mg/mL pembrolizumab, about 0.1-0.6 mg/mL polysorbate 80, about 5-25 mM methionine, about 5-20 mg/mL arginine, about 5-20 mg/mL proline or about 5-20 mg/mL sucrose, and about 1-4000 IU/mL hyaluronidase, wherein pH of the pharmaceutical composition is about 5.0-6.0, for example, the pH of the pharmaceutical composition may be about 5.0, 5.2, 5.5, 5.7, or 6.0, and optionally, nitrogen is introduced as required.

More preferably, the present disclosure provides a pharmaceutical composition of an anti-PD-1 antibody, comprising:
(1) about 155 mg/mL pembrolizumab, about 1.5% arginine, about 1.5% proline, about 10 mM histidine-hydrochloric acid, about 0.4 mg/mL polysorbate 80, about 15 mM methionine, and about 2000 IU/mL hyaluronidase, wherein pH is about 5.5, and optionally, nitrogen is introduced;
(2) about 160 mg/mL pembrolizumab, about 1.5% arginine, about 1.5% proline, about 10 mM histidine-hydrochloric acid, about 0.4 mg/mL polysorbate 80, about 15 mM methionine, and about 2000 IU/mL hyaluronidase, wherein pH is about 5.5, and optionally, nitrogen is introduced;
(3) about 165 mg/mL pembrolizumab, about 1.5% arginine, about 1.5% proline, about 10 mM histidine-hydrochloric acid, about 0.4 mg/mL polysorbate 80, about 15 mM methionine, and about 2000 IU/mL hyaluronidase, wherein pH is about 5.5, and optionally, nitrogen is introduced;
(4) about 170 mg/mL pembrolizumab, about 2% arginine, about 1% proline, about 10 mM histidine-hydrochloric acid, about 0.4 mg/mL polysorbate 80, about 15 mM methionine, and about 2000 IU/mL hyaluronidase, wherein pH is about 5.5, and optionally, nitrogen is introduced;
(5) about 170 mg/mL pembrolizumab, about 1.5% arginine, about 1.5% proline, about 10 mM histidine-hydrochloric acid, about 0.4 mg/mL polysorbate 80, about 15 mM methionine, and about 2000 IU/mL hyaluronidase, wherein pH is about 5.5, and optionally, nitrogen is introduced;
(6) about 170 mg/mL pembrolizumab, about 1% arginine, about 2% proline, about 10 mM histidine-hydrochloric acid, about 0.4 mg/mL polysorbate 80, about 15 mM methionine, and about 2000 IU/mL hyaluronidase, wherein pH is about 5.5, and optionally, nitrogen is introduced;
(7) about 175 mg/mL pembrolizumab, about 1.5% arginine, about 1.5% proline, about 10 mM histidine-hydrochloric acid, about 0.4 mg/mL polysorbate 80, about 15 mM methionine, and about 2000 IU/mL hyaluronidase, wherein pH is about 5.5, and optionally, nitrogen is introduced;
(8) about 180 mg/mL pembrolizumab, about 1.5% arginine, about 1.5% proline, about 10 mM histidine-hydrochloric acid, about 0.4 mg/mL polysorbate 80, about 15 mM methionine, and about 2000 IU/mL hyaluronidase, wherein pH is about 5.5, and optionally, nitrogen is introduced;
(9) about 170 mg/mL pembrolizumab, about 1.5% arginine, about 1.5% proline, about 10 mM histidine-hydrochloric acid, about 0.5 mg/mL poloxamer 188, about 15 mM methionine, and about 2000 IU/mL hyaluronidase, wherein pH is about 5.5, and optionally, nitrogen is introduced;
(10) about 165 mg/mL pembrolizumab, about 2% arginine, about 2% sucrose, about 10 mM histidine-hydrochloric acid, about 0.4 mg/mL polysorbate 80, about 15 mM methionine, and about 2000 IU/mL hyaluronidase, wherein pH is about 5.0, and optionally, nitrogen is introduced;
(11) about 170 mg/mL pembrolizumab, about 2% arginine, about 2% sucrose, about 10 mM histidine-hydrochloric acid, about 0.4 mg/mL polysorbate 80, about 15 mM methionine, and about 2000 IU/mL hyaluronidase, wherein pH is about 5.5, and optionally, nitrogen is introduced;
(12) about 165 mg/mL pembrolizumab, about 2% arginine, about 2% sucrose, about 10 mM histidine-hydrochloric acid, about 0.4 mg/mL polysorbate 80, about 15 mM methionine, and about 2000 IU/mL hyaluronidase, wherein pH is about 5.5, and optionally, nitrogen is introduced.

Meanwhile, the present disclosure further provides a lyophilized formulation, wherein the lyophilized formulation is obtained by lyophilizing the pharmaceutical composition of the present disclosure, or the lyophilized formulation, upon reconstitution, yields the pharmaceutical composition of the present disclosure.

In addition, the present disclosure further provides a method for treating a tumor, the method comprises administering to a subject the pharmaceutical composition or the lyophilized formulation of the present disclosure. The tumor is nasopharyngeal cancer, small cell lung cancer, non-small cell lung cancer, gastric cancer, esophageal cancer, pancreatic cancer, colorectal cancer, melanoma, kidney cancer, breast cancer, liver cancer, ovarian cancer, cervical cancer, bladder cancer, or head and neck tumor.

The present disclosure further provides use of the pharmaceutical composition or the lyophilized formulation in the preparation of a medicament for treating a tumor. The tumor is nasopharyngeal cancer, small cell lung cancer, non-small cell lung cancer, gastric cancer, esophageal cancer, pancreatic cancer, colorectal cancer, melanoma, kidney cancer, breast cancer, liver cancer, ovarian cancer, cervical cancer, bladder cancer, or head and neck tumor.

### BRIEF DESCRIPTION OF THE DRAWINGS

FIG. 1A shows the SEC-HPLC purity of the samples in Example 1.
FIG. 1B shows the nrCE-SDS purity of the samples in Example 1.
FIG. 2 shows the SEC-HPLC purity of the samples in Example 2.
FIG. 3A shows the SEC-HPLC purity of the samples in Example 3.
FIG. 3B shows the IEC-HPLC purity of the samples in Example 3.
FIG. 4 is a graph showing the comparison of the viscosity of the samples in Example 3.
FIG. 5A is a graph showing the distribution of insoluble microparticles in the samples in Example 3 after 10d of light exposure.
FIG. 5B is a graph showing the distribution of insoluble microparticles in the samples in Example 3 after 14d at high temperature.
FIG. 6A shows the SEC-HPLC purity of the samples in Example 4.
FIG. 6B shows the nrCE-SDS purity of the samples in Example 4.
FIG. 6C shows the rCE-SDS purity of the samples in Example 4.

### DETAILED DESCRIPTION

All publications, patents, and patent applications referred to in the specification are incorporated herein by reference to the same extent as if each publication, patent, or patent application was specifically and individually directed to be incorporated by reference.

Before the present disclosure is described in detail below, it will be appreciated that the present disclosure is not limited to the particular methodology, protocols, and reagents described herein, as these may vary. It will also be appreciated that the terms used herein are only intended to describe specific embodiments rather than to limit the scope of the present disclosure. Unless otherwise defined, any technical and scientific term used herein has the same meaning as commonly understood by those of ordinary skill in the art to which the present disclosure pertains.

Certain embodiments disclosed herein encompass numerical ranges, and certain aspects of the present disclosure may be described by means of ranges. Unless otherwise indicated, it will be appreciated that the numerical ranges or the description by means of ranges are merely for the purposes of brevity and convenience, and should not be construed as a strict definition of the scope of the present disclosure. Accordingly, the description by means of ranges should be considered to have specifically disclosed all the possible subranges as well as all the possible specific numerical points within that range, as if those subranges and numerical points were explicitly disclosed herein. The principle described above applies regardless of the extent of the numerical values. When a range description is present, the range includes range endpoints.

The term "about" includes and describes the value or parameter itself. For example, "about x" includes and describes "x" itself. As used herein, the term "about", when used in connection with a measured value or to modify a value, unit, constant, or series of values, refers to a variation of ±1% to 10% in addition to the value or parameter itself. In some embodiments, the term "about", when used in connection with a measured value or to modify a value, unit, constant, or series of values, refers to a variation of ±1%, ±2%, ±3%, ±4%, ±5%, ±6%, ±7%, ±8%, ±9%, or ±10%.

The term "pembrolizumab" is a humanized antibody for cancer immunotherapy, which is currently commercially available under the brand name KEYTRUDA^{®} as a commercial product. "Pembrolizumab", as the common name for the antibody, may also refer only to the active ingredient itself. It comprises two homologous heavy chains and two homologous light chains, wherein the sequence of the heavy chain is shown in SEQ ID NO: 1, and the sequence of the light chain is shown in SEQ ID NO: 2.

The terms "formulation", "pharmaceutical formulation", "formula", and "pharmaceutical formula" are used interchangeably herein, and refer to a form that allows the pharmaceutically active ingredient to exist effectively and does not contain additional components that are toxic to a subject to whom the formulation is to be administered.

The term "pharmaceutical composition" refers to a formulation or a combination of formulations containing one, two, or more active ingredients, which allows the active ingredients contained therein to exist in an effective biologically active form and does not contain additional ingredients having toxicity unacceptable to a subject to whom the formulation is administered. In certain narrow circumstances, for example, when only one pharmaceutically active ingredient is contained, "pharmaceutical composition" and "pharmaceutical formulation" or "pharmaceutical formula" may also be used interchangeably. "Pharmaceutical composition", when present in the form of a combination of separate formulations containing two or more different active ingredients, can be administered simultaneously, sequentially, separately, or intermittently, with the purpose of exerting the biological activity of the multiple active ingredients for jointly treating a disease.

The term "high concentration" refers to a formulation comprising an active pharmaceutical ingredient (API) at a concentration of about 80-200 mg/mL.

The term "pharmaceutically acceptable excipient" refers to those substances/reagents that can be reasonably administered to a subject to provide an effective amount of the active pharmaceutical ingredient in a stable formulation form. Suitable pharmaceutically acceptable excipients are well known in the art and include, but are not limited to, buffer agents, stabilizers, surfactants, and the like.

The term "stabilizer" is a pharmaceutically acceptable excipient that is used to protect an active pharmaceutical ingredient and/or formulation from chemical and/or physical degradation during production, storage, and use.

The term "surfactant" refers to a pharmaceutically acceptable excipient that is used to protect a protein formulation from mechanical stress, such as agitation and shear.

The term "antioxidant" refers to a compound that prevents oxidation of an active ingredient under storage conditions.

The term "diffusing agent" refers to a compound used to increase the permeability of an active substance into tissues (e.g., skin) and facilitate effective diffusion of the active substance. For example, hyaluronidase, which can hydrolyze hyaluronic acid, thereby reducing the viscosity of hyaluronic acid in the extracellular matrix and enabling the pharmaceutical formulation to disperse more readily in tissues.

The term "buffer agent" refers to a reagent that maintains solution pH of a formulation within an acceptable range. The histidine-hydrochloric acid buffer system herein may be prepared with histidine and diluted hydrochloric acid or prepared with histidine and histidine hydrochloride. Meanwhile, the pharmaceutical composition herein may be a self-buffering system without a buffer agent.

The term "self-buffering" means excluding components that are traditionally used to impart buffering capacity in a formulation, for example excluding buffer agents including, but not limited to: a histidine buffer agent, a phosphate buffer agent, a citric acid buffer agent, an acetic acid buffer agent, a carbonic acid buffer agent, a succinic acid buffer agent, a tartaric acid buffer agent, a maleic acid buffer agent, a mixture thereof, or the like. In addition to any buffering capacity present in a formulation without a buffer agent component, it is known that proteins can provide buffering capacity to a formulation. Thus, this does not mean that the formulation cannot have any buffering capacity.

The term "lyophilized formulation" refers to a composition obtained or obtainable by a lyophilization process of a liquid formulation. Preferably, it is a solid composition with a water content of less than 5%, preferably less than 3%.

The term "insoluble microparticle" includes, but is not limited to, microparticles introduced during the product preparation process, fatty acid particles resulting from the degradation of polysorbate, silicone oil, macromolecular particles caused by protein aggregation, and other unknown particles, such as possible debris of packaging materials, crystallization of excipients, and the like, and no degradation of polysorbate is observed herein.

The term "treatment" refers to clinical intervention in an attempt to alter a disease process of an individual or to treat a cell-caused disease process, either prophylactically or during a clinical pathology. Therapeutic effects include, but are not limited to, preventing the occurrence or recurrence of a disease, alleviating a symptom, reducing any direct or indirect pathological consequences of a disease, preventing metastasis, slowing the progression of a disease, ameliorating or relieving a condition, relieving or improving prognosis, or the like.

As used herein, the term "individual", "subject", or "patient" refers to any animal, e.g., a mammal or a marsupial. The individual of the present disclosure includes, but is not limited to, humans, non-human primates (e.g., cynomolgus monkeys, rhesus monkeys, or other types of macaques), mice, pigs, horses, donkeys, cows, sheep, rats, and any species of poultry.

The term "disease", "condition", "disorder", or the like refers to any alteration or dysregulation that impairs or interferes with the normal function of cells, tissues, or organs. For example, the "disease" includes, but is not limited to: tumors, pathogen infections, autoimmune diseases, T cell dysfunctional diseases, defects in immune tolerance (e.g., transplant rejection), or the like.

The term "tumor" refers to a disease characterized by pathological proliferation of cells or tissues and subsequent migration thereof to or invasion thereof of other tissues or organs. Tumor growth is generally uncontrolled and progressive, without inducing or inhibiting normal cell proliferation. Tumors include "cancer", which generally refers to all malignant tumors.

### EXAMPLES

### Experimental Example 1. Size Exclusion Chromatography (SEC-HPLC)

Aggregates, monomers, and fragments were quantified by size exclusion chromatography. The method utilized an X Bridge BEH 200Å SEC, 7.8 × 300 mm chromatographic column and was run on a Waters e2695-2489 HPLC system. The mobile phase was 100 mM sodium phosphate and 150 mM sodium chloride, with the pH being 6.8. The samples were diluted to 1 mg/mL with the mobile phase, and the injection volume was 20 µL. The isocratic elution was performed at a flow rate of 0.5 mL/min for 30 min, and the detection wavelength was 215 nm. The integration treatment was performed using Empower 3 software, and the percentage content of each component was calculated by area normalization.

### Experimental Example 2. Capillary Electrophoresis (CE-SDS) Assay

The non-reduced purity and reduced purity of the product were assayed by capillary electrophoresis (CE-SDS). The assay was performed on a SCIEX PA800 plus capillary electrophoresis system with a 50 µM I.D. uncoated quartz capillary, with a capillary effective separation length of 20 cm, and a total length of 30.2 cm. Determination was performed using PDA at 220 nm with a bandwidth of 10 nm. The reduced purity was assayed with injection at 5 kV for 20 s followed by separation at 15 kV for 30 min. The non-reduced purity was assayed with injection at 10 kV for 20 s followed by separation at 15 kV for 40 min. The integration treatment was performed using 32Karat software.

### Experimental Example 3. Ion Exchange Chromatography (IEC-HPLC) Assay

The charge heterogeneity of the product was assayed by ion exchange chromatography. Various post-translational modifications of recombinant protein drugs can alter the surface charge distribution properties of proteins, resulting in their charge heterogeneity. The method utilized a weak cation exchange chromatographic column ProPac WCX-10 (4.0 mm × 250 mm, 10 µm, Thermo) and was run on a Waters e2695-2489 HPLC system. Mobile phase A: 24 mM MES, 4% acetonitrile (v/v), pH 6.0; mobile phase B: 20 mM sodium phosphate, 95 mM NaCl, 4% acetonitrile (v/v), pH 8.0; samples were diluted to 8 mg/mL with ultrapure water, and the injection volume was 10 µL. The elution was performed at a flow rate of 0.5 mL/min, and the detection wavelength was 280 nm. The integration treatment was performed using Empower 3 software, and the percentage content of each component was calculated by area normalization.

### Experimental Example 4. Method for Insoluble Microparticle Assay (FlowCam)

The insoluble microparticles of the product were assayed using a flow imaging microscope FlowCam 8100 with the following parameter settings: a sample loading volume of 200 µL and a flow rate of 0.05 mL/min; the sample test was performed, and the analysis software Visual Spreadsheet was used.

### Experimental Example 5. Protein Content Assay

The protein content was assayed using a Lunatic microvolume spectrophotometer. A blank correction with purified water was applied, and ultraviolet light absorption at 280 nm was corrected by light scattering at 330 nm. The protein concentration was calculated according to equation 1, where the extinction coefficient ε was 1.42 (L/g·cm⁻¹). $Protein content = \frac{A \left(280\right) - A \left(330\right)}{ε}$ Equation 1

### Experimental Example 6. Viscosity Assay

The viscosity of the samples was measured using an m-VROC microfluidic rheometer at 25 °C.

The present disclosure is further illustrated in detail by the following examples. Changes that alter the concentrations of certain substances or the addition of other substances without significantly improving protein stability on the basis of the present disclosure are still considered part of the present disclosure.

### Example 1. Effect of Antioxidants on Light Exposure Stability of Formulas

To prepare the formulation formula solution, the antibody was adjusted to the desired concentration by ultrafiltration concentration. After completion of the ultrafiltration concentration, the excipients to be added were added to the antibody solution, and finally, the protein concentration was adjusted to about 170 mg/mL. All formula samples were individually sterilized and filtered through a 0.22 µm filter and placed into sterile 2 mL tubular vials under controlled conditions. Nitrogen was introduced as required, and the vials were sealed with a coated rubber stopper and an aluminum-plastic combination cap. These formulations were examined for stability under high temperature (40 °C ± 2 °C) and light exposure (illuminance: 5000 lx, ultraviolet: 90 µw/cm², 25 °C ± 2 °C) conditions. Tables 1-2 summarize the different formulation formulas and stability results:

**Table 1. Formula composition**

| Formula | Pembrolizumab | Stabilizer | Buffer agent | Surfactant | Antioxidant | pH |
|---|---|---|---|---|---|---|
| R1 | 170 mg/ml | 7% sucrose | 10 mM histidine-hydrochloric acid | 0.2 mg/mL polysorbate 80 | Nitrogen introduction | pH 5.5 |
| R2 | 170 mg/ml | 7% sucrose | 10 mM histidine-hydrochloric acid | 0.2 mg/mL polysorbate 80 | 15 mM Met | pH 5.5 |
| R3 | 170 mg/ml | 7% sucrose | 10 mM histidine-hydrochloric acid | 0.5 mg/mL poloxamer 188 | 15 mM Met | pH 5.5 |
| R4 | 170 mg/ml | 7% sucrose | 10 mM histidine-hydrochloric acid | 0.2 mg/mL polysorbate 80 | Nitrogen introduction | pH 5.5 |
| R5 | 170 mg/ml | 7% sucrose | 10 mM histidine-hydrochloric acid | 0.5 mg/mL poloxamer 188 | 15 mM Met, nitrogen introduction | pH 5.5 |

**Table 2. Stability data**

| Formula | Investigation condition | SEC-HPLC (%) | | | nrCE-SDS (%) | | |
|---|---|---|---|---|---|---|---|
| | | HMWS | MP | LMWS | HMWS | MP | LMWS |
| R1 | 0d | 0.28 | 99.7 | 0.02 | / | 99.5 | 0.49 |
| | Light exposure 10d | 3.7 | 95.4 | 0.82 | 1.5 | 94.5 | 4.0 |
| | 40 °C 14d | 0.70 | 99.2 | 0.08 | 0.52 | 98.4 | 1.0 |
| R2 | 0d | 0.28 | 99.7 | 0.02 | / | 99.5 | 0.49 |
| | Light exposure 10d | 5.1 | 94.8 | 0.14 | 2.3 | 94.4 | 3.3 |
| | 40 °C 14d | 0.67 | 99.3 | 0.07 | 0.54 | 98.4 | 1.1 |
| R3 | 0d | 0.28 | 99.7 | 0.02 | / | 99.5 | 0.49 |
| | Light exposure 10d | 4.6 | 95.3 | 0.13 | 2.3 | 94.6 | 3.1 |
| | 40 °C 14d | 0.68 | 99.2 | 0.08 | 0.50 | 98.5 | 1.0 |
| R4 | 0d | 0.28 | 99.7 | 0.02 | / | 99.5 | 0.49 |
| | Light exposure 10d | 3.3 | 94.8 | 1.5 | 1.6 | 94.5 | 4.0 |
| | 40 °C 14d | 0.67 | 99.3 | 0.07 | 0.42 | 98.5 | 1.1 |
| R5 | 0d | 0.28 | 99.7 | 0.02 | / | 99.5 | 0.49 |
| | Light exposure 10d | 2.5 | 96.8 | 0.69 | 0.99 | 95.6 | 3.4 |
| | 40 °C 14d | 0.71 | 99.2 | 0.07 | 0.38 | 98.6 | 1.0 |

| | | | | | | | |
|---|---|---|---|---|---|---|---|
| Note: / indicates not detected; for the data on 0d, reference was made to the assay results of formula R2. | | | | | | | |

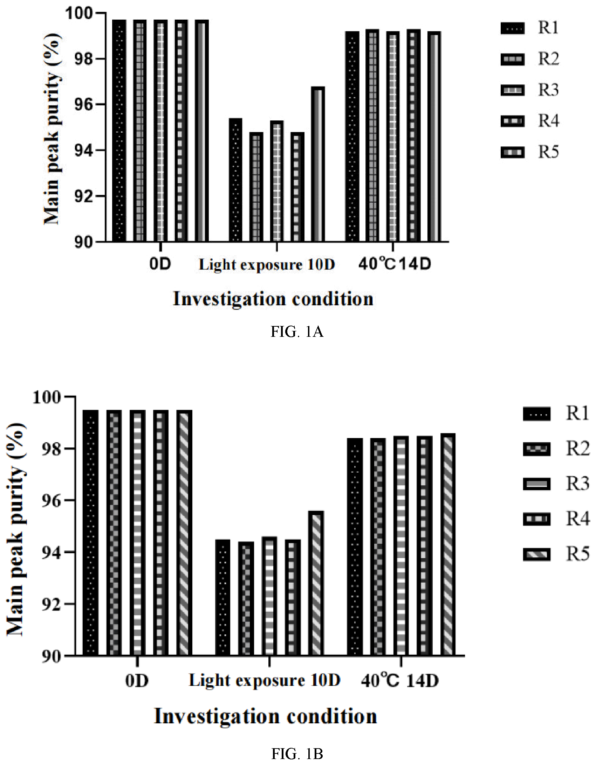

According to the data in Table 2 and FIGs. 1A and 1B, it can be seen that: after 10 days of light exposure, formula R5 exhibited the SEC-HPLC main peak purity higher by 2.0% and 1.5%, respectively, and the nrCE-SDS main peak purity higher by 1.2% and 1.0%, respectively, as compared with formulas R2 and R3, each containing only 15 mM methionine. The test results show that adding a nitrogen introduction condition while adding methionine can effectively prevent the formation of aggregates under the light exposure conditions.

### Example 2. Stability of Antibody Formulations in Amino Acid System

To prepare the formulation formula solution, the antibody was adjusted to the desired concentration by ultrafiltration concentration. After completion of the ultrafiltration concentration, the excipients to be added were added to the antibody solution, and finally, the protein concentration was adjusted to about 170 mg/mL. All formulas were individually sterilized and filtered through a 0.22 µm filter and placed into sterile 2 mL tubular vials under controlled conditions. The vials were sealed with a coated rubber stopper and an aluminum-plastic combination cap. These formulations were examined for stability under the high temperature (40 °C ± 2 °C) condition. Tables 3-4 show the different formulation formulas and stability results:

**Table 3. Formula composition**

| Formula | Pembrolizumab | Formulation system | pH | Hyaluronidase |
|---|---|---|---|---|
| R6 | 170 mg/ml | 7% sucrose, 10 mM histidine-hydrochloric acid, 10 mM methionine, 0.02% polysorbate 80 | pH 5.5 | 2000 IU/ml |
| R7 | 170 mg/ml | 8% trehalose, 10 mM histidine-hydrochloric acid, 10 mM methionine, 0.02% polysorbate 80 | pH 5.5 | 2000 IU/ml |
| R8 | 170 mg/ml | 2.4% proline, 10 mM histidine-hydrochloric acid, 10 mM methionine, 0.02% polysorbate 80 | pH 5.5 | 2000 IU/ml |
| R9 | 170 mg/ml | 3% arginine, 10 mM histidine-hydrochloric acid, 10 mM methionine, 0.02% polysorbate 80 | pH 5.5 | 2000 IU/ml |

**Table 4. Stability data**

| Formula | Investigation condition | Protein concentration (mg/mL) | Viscosity (cP) | SEC-HPLC (%) | | |
|---|---|---|---|---|---|---|
| | | | | HMWS | MP | LMWS |
| R6 | 0d | 176.7 | 23.7 | 0.2 | 99.7 | 0.05 |
| | 40 °C 14d | 179.5 | 25.8 | 0.65 | 99.3 | 0.08 |
| | 40 °C 30d | NA | NA | 0.96 | 98.9 | 0.12 |
| R7 | 0d | 167.1 | 18.9 | 0.2 | 99.7 | 0.05 |
| | 40 °C 14d | 171.3 | 21.4 | 0.65 | 99.3 | 0.09 |
| | 40 °C 30d | NA | NA | 0.99 | 98.9 | 0.13 |
| R8 | 0d | 172.5 | 17.3 | 0.2 | 99.7 | 0.05 |
| | 40 °C 14d | 172.7 | 18.0 | 0.60 | 99.3 | 0.09 |
| | 40 °C 30d | NA | NA | 0.85 | 99.0 | 0.14 |
| R9 | 0d | 167.5 | 11.7 | 0.2 | 99.7 | 0.05 |
| | 40 °C 14d | 172.5 | 12.4 | 0.43 | 99.5 | 0.10 |
| | 40 °C 30d | NA | NA | 0.61 | 99.2 | 0.15 |

| | | | | | | |
|---|---|---|---|---|---|---|
| Note: NA indicates not detected. | | | | | | |

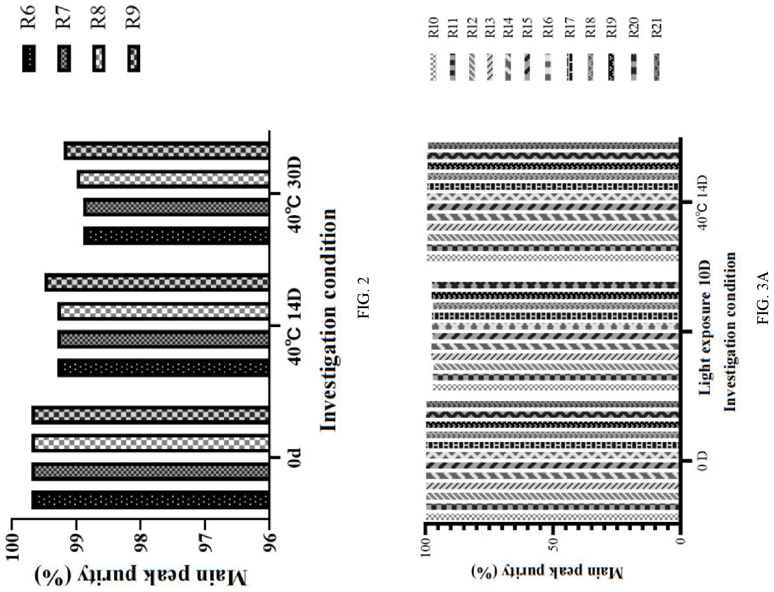

According to the data in Table 4 and FIG. 2, it can be seen that: the viscosity of the product of formulation formula R8 was reduced by about 27% and the viscosity of the product of formulation formula R9 was reduced by about 50% as compared with formula R6. After storage at high temperature (40 °C, 30d), the SEC-HPLC main peak purity of formulation formula R8 was not greatly different from that of formula R6, and the SEC-HPLC main peak purity of formula R9 was slightly superior by 0.3%. The results show that the amino acid system in the formulations can effectively reduce the viscosity of the pembrolizumab high-concentration formulations, while the formulations have excellent thermal stability, with 3% arginine being preferred.

### Example 3. Stability of Pembrolizumab Antibody Formulations with Different Amino Acid Ratios

To prepare the formulation formula solution, the antibody was adjusted to the desired concentration by ultrafiltration concentration. After completion of the ultrafiltration concentration, the excipients to be added were added to the antibody solution, and finally, the protein concentration was adjusted to the desired concentration. All formulas were individually sterilized and filtered through a 0.22 µm filter and placed into sterile 2 mL tubular vials under controlled conditions. The vials were sealed with a coated rubber stopper and an aluminum-plastic combination cap. These formulations were examined for stability under high temperature (40 °C ± 2 °C) and light exposure (illuminance: 5000 lx, ultraviolet: 90 µw/cm², 25 °C ± 2 °C) conditions. Tables 5-8 show the different formulation formulas and stability results:

**Table 5. Formula composition**

| Formula | Pembrolizumab | Stabilizer | Buffer agent | Surfactant | Antioxidant | Hyaluronidase | pH |
|---|---|---|---|---|---|---|---|
| R10 | 165 mg/ml | 2% sucrose + 2% L-arginine | 10 mM histidine-hydrochloric acid | 0.04% polysorbate 80 | 15 mM Met | 2000 IU/ml | pH5.5 |
| R11 | 165 mg/ml | 1.5% proline + 1.5% L-arginine | 10 mM histidine-hydrochloric acid | 0.04% polysorbate 80 | 15 mM Met | 2000 IU/ml | pH5.5 |
| R12 | 170 mg/ml | 7% sucrose | 10 mM histidine-hydrochloric acid | 0.04% polysorbate 80 | 15 mM Met | 2000 IU/ml | pH5.5 |
| R13 | 170 mg/ml | 3% L-arginine | 10 mM histidine-hydrochloric acid | 0.04% polysorbate 80 | 15 mM Met | 2000 IU/ml | pH5.5 |
| R14 | 170 mg/ml | 2% proline + 1% L-arginine | 10 mM histidine-hydrochloric acid | 0.04% polysorbate 80 | 15 mM Met | 2000 IU/ml | pH5.5 |
| R15 | 170 mg/ml | 1.5% proline + 1.5% L-arginine | 10 mM histidine-hydrochloric acid | 0.04% polysorbate 80 | 15 mM Met | 2000 IU/ml | pH5.5 |
| R16 | 170 mg/ml | 1% proline + 2% L-arginine | 10 mM histidine-hydrochloric acid | 0.04% polysorbate 80 | 15 mM Met | 2000 IU/ml | pH5.5 |
| R17 | 170 mg/ml | 3% proline | 10 mM histidine-hydrochloric acid | 0.04% polysorbate 80 | 15 mM Met | 2000 IU/ml | pH5.5 |
| R18 | 170mg/ml | 2.5% phenylalanine | 10 mM histidine-hydrochloric acid | 0.04% polysorbate 80 | 15 mM Met | 2000 IU/ml | pH5.5 |
| R19 | 170 mg/ml | / | 10 mM histidine-hydrochloric acid | 0.04% polysorbate 80 | 15 mM Met | 2000 IU/ml | pH5.5 |
| R20 | 170 mg/ml | 2% sucrose + 2% L-arginine | 10 mM histidine-hydrochloric acid | 0.04% polysorbate 80 | 15 mM Met | 2000 IU/ml | pH5.5 |
| R21 | 180 mg/ml | 7% sucrose | / | 0.02% polysorbate 80 | 10 mM Met | 2000 IU/ml | pH5.5 |

**Table 6. Stability results**

| Formula | Investigation condition | Protein concentration (mg/mL) | Viscosity (cP) | SEC-HPLC (%) | | | IEC-HPLC (%) | | |
|---|---|---|---|---|---|---|---|---|---|
| | | | | HMWS | MP | LMWS | ACIDIC | MP | BASIC |
| R10 | 0h | NA | NA | 0.13 | 99.9 | 0.01 | 14.8 | 73.9 | 11.3 |
| | Light exposure 10d | NA | NA | 2.8 | 97.1 | 0.09 | 19.0 | 52.1 | 29.0 |
| | 40 °C 14d | NA | NA | 0.37 | 99.5 | 0.09 | 22.3 | 66.0 | 11.7 |
| R11 | 0h | NA | NA | 0.15 | 99.8 | 0.01 | 14.9 | 73.9 | 11.3 |
| | Light exposure 10d | NA | NA | 2.7 | 97.2 | 0.08 | 19.2 | 51.8 | 29.0 |
| | 40 °C 14d | NA | NA | 0.40 | 99.5 | 0.09 | 22.5 | 65.9 | 11.6 |
| R12 | 0d | 176.2 | 27.7 | 0.24 | 99.8 | 0.01 | 14.9 | 71.4 | 13.7 |
| | Light exposure 10d | 179.9 | NA | 3.1 | 96.8 | 0.09 | 19.2 | 50.0 | 30.7 |
| | 40 °C 14d | 174.5 | 25.8 | 0.77 | 99.2 | 0.05 | 23.5 | 62.3 | 14.3 |
| R13 | 0d | 169.1 | 12.4 | 0.24 | 99.8 | 0.01 | 14.9 | 71.4 | 13.7 |
| | Light exposure 10d | 168.4 | NA | 2.1 | 97.8 | 0.08 | 17.7 | 52.9 | 29.4 |
| | 40 °C 14d | 176.1 | 12.1 | 0.50 | 99.4 | 0.06 | 22.1 | 63.7 | 14.2 |
| R14 | 0d | 167.6 | 12.6 | 0.24 | 99.8 | 0.01 | 14.9 | 71.4 | 13.7 |
| | Light exposure 10d | 164.1 | NA | 2.2 | 97.7 | 0.08 | 18.2 | 52.7 | 29.1 |
| | 40 °C 14d | 171.7 | 12.6 | 0.55 | 99.4 | 0.06 | 23.0 | 62.9 | 14.1 |
| R15 | 0d | 172.0 | 13.7 | 0.24 | 99.8 | 0.01 | 14.9 | 71.4 | 13.7 |
| | Light exposure 10d | 174.2 | NA | 2.6 | 97.3 | 0.10 | 18.4 | 50.6 | 31.0 |
| | 40 °C 14d | 177.4 | 12.0 | 0.53 | 99.4 | 0.06 | 22.9 | 63.1 | 14.1 |
| R16 | 0d | 166.5 | 12.9 | 0.24 | 99.8 | 0.01 | 14.9 | 71.4 | 13.7 |
| | Light exposure 10d | 176.1 | NA | 2.4 | 97.5 | 0.11 | 18.1 | 51.6 | 30.4 |
| | 40 °C 14d | 173.5 | 13.2 | 0.54 | 99.4 | 0.08 | 22.6 | 63.2 | 14.1 |
| R17 | 0d | 167.4 | 15.3 | 0.24 | 99.8 | 0.01 | 14.9 | 71.4 | 13.7 |
| | Light exposure 10d | 160.4 | NA | 2.4 | 97.5 | 0.08 | 19.1 | 52.3 | 28.6 |
| | 40 °C 14d | 169.5 | 15.7 | 0.68 | 99.3 | 0.05 | 23.8 | 62.4 | 13.8 |
| R18 | 0d | 179.9 | 14.5 | 0.24 | 99.8 | 0.01 | 14.9 | 71.4 | 13.7 |
| | Light exposure 10d | 165.8 | NA | 2.7 | 97.2 | 0.09 | 18.4 | 54.8 | 26.8 |
| | 40 °C 14d | 167.6 | 13.5 | 0.65 | 99.3 | 0.06 | 23.5 | 62.7 | 13.9 |
| R19 | 0d | 168.7 | 17.3 | 0.24 | 99.8 | 0.01 | 14.9 | 71.4 | 13.7 |
| | Light exposure 10d | 169.3 | NA | 2.3 | 97.6 | 0.07 | 18.2 | 51.0 | 30.8 |
| | 40 °C 14d | 166.5 | 17.5 | 0.70 | 99.2 | 0.06 | 22.5 | 63.2 | 14.3 |
| R20 | 0d | 165.3 | 13.9 | 0.24 | 99.8 | 0.01 | 14.9 | 71.4 | 13.7 |
| | Light exposure 10d | 170.2 | NA | 2.4 | 97.5 | 0.10 | 19.2 | 50.0 | 30.7 |
| | 40 °C 14d | 173.0 | 13.6 | 0.52 | 99.4 | 0.07 | 23.5 | 62.3 | 14.3 |
| R21 | 0d | 178.1 | 27.5 | 0.2 | 99.7 | 0.05 | 14.4 | 71.4 | 14.2 |
| | 40 °C 14d | 181.5 | 29.0 | 0.85 | 99.1 | 0.06 | 22.0 | 63.7 | 14.4 |

| | | | | | | | | | |
|---|---|---|---|---|---|---|---|---|---|
| Note: NA indicates not detected. | | | | | | | | | |

**Table 7. Insoluble microparticle results after 10 days of light exposure**

| Sample | Insoluble microparticle after 10d of light exposure (particles/mL) | | | | | | | Morphology (particles/mL) | | | | |
|---|---|---|---|---|---|---|---|---|---|---|---|---|
| | | | | | | | | Silicone oil | | Bubble | Non-spherical particle | |
| | All | ≥2µm | ≥5µm | ≥10µm | ≥25µm | ≥50µm | ≥100µm | 2-10µm | ≥10µm | | 2-10µm | ≥10µm |
| R12 | 54704 | 35130 | 4602 | 889 | 65 | 9 | 0 | 24657 | 37 | 9 | 9593 | 852 |
| R13 | 20905 | 10880 | 1856 | 442 | 128 | 0 | 0 | 6815 | 10 | 0 | 3623 | 432 |
| R14 | 20697 | 12531 | 1854 | 479 | 63 | 0 | 0 | 8864 | 63 | 0 | 3177 | 417 |
| R15 | 15486 | 5936 | 967 | 346 | 20 | 0 | 0 | 3217 | 10 | 10 | 2352 | 326 |
| R16 | 15793 | 7830 | 1101 | 278 | 62 | 10 | 0 | 5031 | 0 | 0 | 2531 | 268 |
| R17 | 11349 | 6293 | 544 | 83 | 9 | 0 | 0 | 3580 | 0 | 0 | 2630 | 74 |
| R18 | 31489 | 20729 | 3459 | 979 | 289 | 56 | 0 | 13204 | 84 | 0 | 6537 | 895 |
| R19 | 66404 | 42588 | 4625 | 652 | 39 | 0 | 0 | 31459 | 49 | 0 | 10516 | 604 |
| R20 | 26627 | 13771 | 1559 | 372 | 50 | 10 | 0 | 9275 | 40 | 0 | 4155 | 332 |

**Table 8. Insoluble microparticle results after 14 days of high temperature (40 °C ± 2 °C)**

| Sample | Insoluble microparticles after 14 days of high temperature (particles/mL) | | | | | | | Morphology (particles/mL) | | | | |
|---|---|---|---|---|---|---|---|---|---|---|---|---|
| | | | | | | | | Silicone oil | | Bubble | Non-spherical particle | |
| | All | ≥2µm | ≥5µm | ≥10µm | ≥25µm | ≥50µm | ≥100µm | 2-10µm | ≥10µm | | 2-10µm | ≥10µm |
| R12 | 35536 | 18756 | 2777 | 652 | 59 | 0 | 0 | 12936 | 20 | 0 | 5168 | 632 |
| R13 | 14347 | 11202 | 1711 | 194 | 22 | 0 | 0 | 8717 | 22 | 0 | 2292 | 172 |
| R14 | 22354 | 13918 | 3265 | 1088 | 114 | 10 | 0 | 8063 | 52 | 0 | 4767 | 1036 |
| R15 | 8130 | 4577 | 652 | 100 | 10 | 0 | 0 | 3021 | 20 | 0 | 1455 | 80 |
| R16 | 11535 | 6906 | 996 | 176 | 0 | 0 | 0 | 4669 | 20 | 0 | 2061 | 156 |
| R17 | 16907 | 10361 | 1375 | 310 | 31 | 0 | 0 | 7621 | 21 | 0 | 2420 | 290 |
| R18 | 17805 | 10571 | 1254 | 309 | 29 | 10 | 0 | 7398 | 10 | 19 | 2855 | 280 |
| R19 | 15842 | 8907 | 1191 | 238 | 10 | 0 | 0 | 6802 | 48 | 0 | 1858 | 191 |
| R20 | 9133 | 5142 | 751 | 205 | 20 | 0 | 0 | 3581 | 39 | 0 | 1356 | 166 |

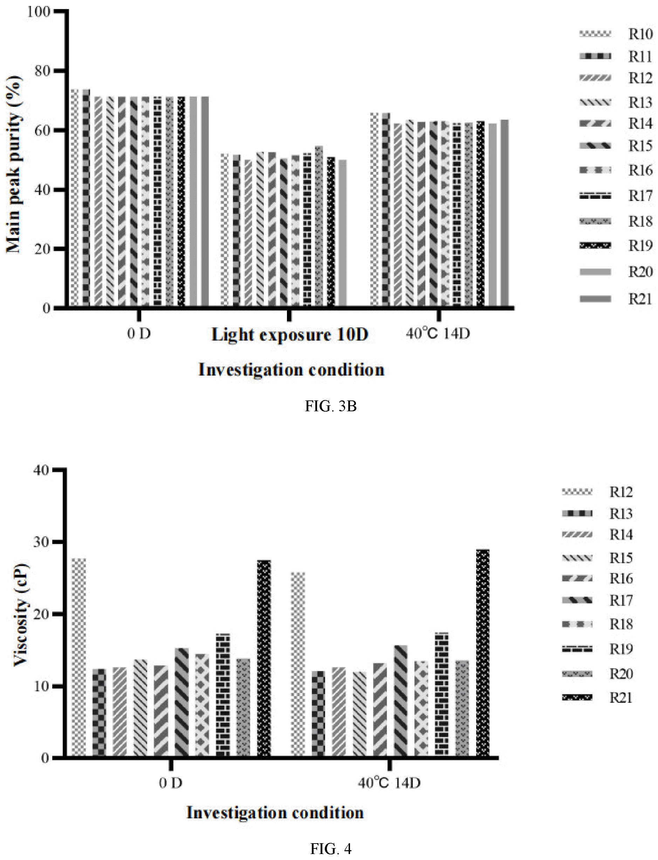

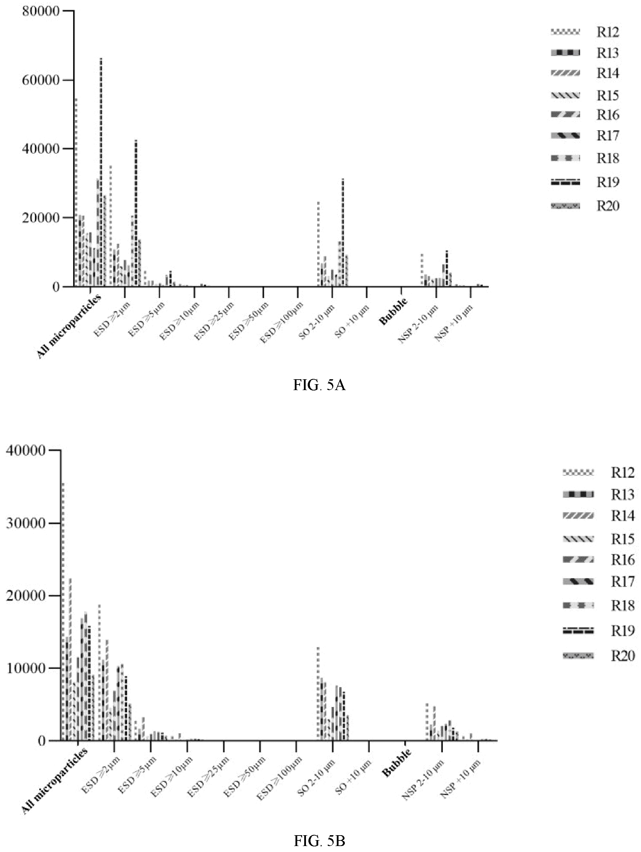

According to the data in Tables 6, 7, and 8 and FIGs. 3A, 3B, 4, 5A, and 5B, it can be seen that: compared with formula R12 containing sucrose, the samples containing the amino acid system and the samples containing the self-buffering system showed no significant difference in the SEC-HPLC main peak purity after storage at 40 °C for 14 days; the SEC-HPLC main peaks of formulas R13, R14, R15, R16, R17, and R20 were reduced by about 2.0%, 2.2%, 2.5%, 2.3%, 2.3%, and 2.3%, after storage under light exposure for 10 days, and the reduction was smaller than that of formula R12 (about 3.0% reduction in the SEC-HPLC main peak). Meanwhile, the viscosity test results show that the viscosity of the samples of formulation formulas R13, R14, R15, R16, R17, and R20 was significantly lower than that of formula R12. The results described above show that arginine and proline played an excellent role in the SEC-HPLC stability in the pembrolizumab formulations, the effect was significantly better than that of sucrose, and the sample viscosity was significantly reduced.

The data results of insoluble microparticles show that after 10 days of light exposure, as compared with the sucrose system (R12), the total numbers of insoluble microparticles of the formulation formula containing 1% arginine and 2% proline (R14), the formulation formula containing 1.5% arginine and 1.5% proline (R15), the formulation formula containing 2% arginine and 1% proline (R16), and the formulation formula containing 2% arginine and 2% sucrose (R20) were about 37.8% (20697/54704), 28.3% (15486/54704), 28.9% (15793/54704), and 48.7% (26627/54704), respectively, of that of formula R12. After 14 days of high temperature treatment, as compared with formula R12, the total numbers of insoluble microparticles of formulas R14, R15, R16, and R20 were about 62.9% (22354/35536), 22.9% (8130/35536), 32.5% (11535/35536), and 25.7% (9133/35536), respectively, of that of formula R12. It should be noted that formulas R15 and R20 have superior effects in terms of insoluble microparticles, as compared with the formulation formula containing only arginine (R13).

Based on the experimental results described above, as compared with formula R12, formulas R15 and R20 have significant advantages in terms of purity, viscosity, and the total numbers of insoluble microparticles, and meanwhile, formulation formula containing 1.5% arginine and 1.5% proline (R11) and the formulation formula containing 2% arginine and 2% sucrose (R10) also achieve excellent effects similar to those of formulas R15 and R20.

### Example 4. Stability of Pembrolizumab Antibody Formulations with Different Excipient Concentrations

To prepare the formulation formula solution, the antibody was adjusted to the desired concentration by ultrafiltration concentration. After completion of the ultrafiltration concentration, the excipients to be added were added to the antibody solution, and finally, the protein concentration was adjusted to the protein concentration in Table 9. All formulas were individually sterilized and filtered through a 0.22 µm filter and placed into sterile 2 mL tubular vials under controlled conditions. The vials were sealed with a coated rubber stopper and an aluminum-plastic combination cap. These formulations were examined for stability under high temperature (40 °C ± 2 °C) and light exposure (illuminance: 5000 lx, ultraviolet: 90 µw/cm², 25 °C ± 2 °C) conditions. Tables 9-11 show the different formulation formulas and stability results:

**Table 9. Formula composition**

| Formula | Pembrolizumab | Stabilizer | Buffer agent | Surfactant | Antioxidant | Hyaluronidase | pH |
|---|---|---|---|---|---|---|---|
| R22 | 165 mg/ml | 7% sucrose | None | 0.02% polysorbate 80 | 10 mM Met | 2000 IU/ml | pH5.5 |
| R23 | 165 mg/ml | 7% sucrose | 10 mM histidine-hydrochloric acid | 0.04% polysorbate 80 | 15 mM Met | 2000 IU/ml | pH5.5 |
| R24 | 80 mg/ml | 1.5% proline + 1.5% L-arginine | 30 mM histidine-hydrochloric acid | 0.01% polysorbate 80 | 5 mM Met | 150 IU/ml | pH5.5 |
| R25 | 200 mg/ml | 1.5% proline + 1.5% L-arginine | 10 mM histidine-hydrochloric acid | 0.1% polysorbate 80 | 15 mM Met | 2000 IU/ml | pH5.5 |
| R26 | 165 mg/ml | 0.1% proline + 0.1% L-arginine | 10 mM histidine-hydrochloric acid | 0.04% polysorbate 20 | 50 mM Met | 10000 IU/ml | pH5.5 |
| R27 | 165 mg/ml | 5% proline + 5% L-arginine | 10 mM histidine-hydrochloric acid | 0.04% polysorbate 80 | 15 mM Met | 2000 IU/ml | pH5.5 |
| R28 | 165 mg/ml | 2% sucrose + 2% L-arginine | 10 mM histidine-hydrochloric acid | 0.04% polysorbate 80 | 15 mM Met | 2000 IU/ml | pH5.0 |
| R29 | 165 mg/ml | 1% sucrose + 0.1% L-arginine | 10 mM histidine-hydrochloric acid | 0.04% polysorbate 80 | 15 mM Met | 2000 IU/ml | pH5.5 |
| R30 | 165 mg/ml | 5% sucrose + 5% L-arginine | 10 mM histidine-hydrochloric acid | 0.04% polysorbate 80 | 15 mM Met | 2000 IU/ml | pH6.0 |

**Table 10. Stability results**

| Formula | Investigation condition | SEC-HPLC(%) | | |
|---|---|---|---|---|
| | | HMWS | Monomer | LMWS |
| R22 | 0h | 0.20 | 99.7 | 0.05 |
| | 40 °C 2W | 0.85 | 99.1 | 0.06 |
| R23 | 0h | 0.74 | 99.3 | / |
| | Light exposure 10d | 4.1 | 95.8 | 0.07 |
| | 40 °C 2W | 0.76 | 99.2 | 0.06 |
| R24 | 0h | 0.45 | 99.5 | / |
| | Light exposure 10d | 2.2 | 97.7 | 0.08 |
| | 40 °C 2W | 0.39 | 99.5 | 0.09 |
| R25 | 0h | 0.75 | 99.2 | 0.02 |
| | Light exposure 10d | 4.5 | 95.4 | 0.10 |
| | 40 °C 2W | 0.74 | 99.2 | 0.09 |
| R26 | 0h | 0.51 | 99.5 | 0.01 |
| | Light exposure 10d | 3.0 | 96.9 | 0.07 |
| | 40 °C 2W | 0.62 | 99.3 | 0.08 |
| R27* | 0h | 0.51 | 99.5 | 0.01 |
| | Light exposure 10d | 3.0 | 96.9 | 0.10 |
| | 40 °C 2W | 0.58 | 99.3 | 0.08 |
| R28* | 0h | 0.51 | 99.5 | 0.01 |
| | Light exposure 10d | 4.3 | 95.6 | 0.10 |
| | 40 °C 2W | 0.97 | 98.9 | 0.13 |
| R29* | 0h | 0.51 | 99.5 | 0.01 |
| | Light exposure 10d | 4.5 | 95.4 | 0.08 |
| | 40 °C 2W | 0.74 | 99.2 | 0.10 |
| R30* | 0h | 0.51 | 99.5 | 0.01 |
| | Light exposure 10d | 2.8 | 97.2 | 0.09 |
| | 40 °C 2W | 0.30 | 99.6 | 0.11 |

| | | | | |
|---|---|---|---|---|
| Note: / indicates not detected; * indicates that the data at 0h of R26 was used as a 0h control. | | | | |

**Table 11. Stability results**

| Formula | Investigation condition | nrCE | | | rCE | | | |
|---|---|---|---|---|---|---|---|---|
| | | HMWS% | Purity% | LMWS% | HMWS% | Purity% | LMWS% | NGHC% |
| R23 | 0h | 0.03 | 99.3 | 0.72 | 0.62 | 98.2 | 1.2 | 0.73 |
| | Light exposure 10d | 1.9 | 95.5 | 2.6 | 1.3 | 97.4 | 1.4 | 0.77 |
| | 40 °C 2W | 0.07 | 98.3 | 1.6 | 0.67 | 97.9 | 1.5 | 0.74 |
| R24 | 0h | / | 98.7 | 1.3 | 0.53 | 98.1 | 1.4 | 0.79 |
| | Light exposure 10d | 1.5 | 95.1 | 3.4 | 1.1 | 97.4 | 1.5 | 0.72 |
| | 40 °C 2W | 0.08 | 98.1 | 1.8 | 0.73 | 97.8 | 1.5 | 0.74 |
| R25 | 0h | 0.02 | 99.1 | 0.85 | 0.60 | 98.2 | 1.2 | 0.82 |
| | Light exposure 10d | 1.9 | 95.1 | 3.0 | 1.2 | 97.4 | 1.4 | 0.86 |
| | 40 °C 2W | 0.11 | 98.3 | 1.6 | 0.73 | 97.6 | 1.7 | 0.79 |
| R26 | 0h | / | 99.4 | 0.59 | 0.58 | 98.2 | 1.2 | 0.77 |
| | Light exposure 10d | 1.3 | 96.3 | 2.3 | 1.1 | 97.5 | 1.4 | 0.71 |
| | 40 °C 2W | 0.09 | 98.7 | 1.3 | 0.63 | 97.8 | 1.6 | 0.71 |
| R27 | 0h | / | 99.4 | 0.59 | 0.58 | 98.2 | 1.2 | 0.77 |
| | Light exposure 10d | 1.4 | 94.5 | 4.1 | 1.2 | 97.2 | 1.6 | 0.80 |
| | 40 °C 2W | 0.10 | 97.4 | 2.5 | 0.56 | 97.8 | 1.7 | 0.82 |
| R28* | 0h | / | 99.4 | 0.59 | 0.58 | 98.2 | 1.2 | 0.77 |
| | Light exposure 10d | 1.7 | 95.4 | 2.9 | 1.2 | 97.3 | 1.5 | 0.95 |
| | 40 °C 2W | 0.13 | 98.3 | 1.6 | 0.60 | 97.6 | 1.9 | 0.90 |
| R29* | 0h | / | 99.4 | 0.59 | 0.58 | 98.2 | 1.2 | 0.77 |
| | Light exposure 10d | 1.7 | 95.7 | 2.6 | 1.1 | 97.5 | 1.4 | 0.73 |
| | 40 °C 2W | 0.11 | 98.5 | 1.4 | 0.71 | 97.8 | 1.5 | 0.69 |
| R30* | 0h | / | 99.4 | 0.59 | 0.58 | 98.2 | 1.2 | 0.77 |
| | Light exposure 10d | 1.3 | 96.2 | 2.6 | 1.2 | 97.3 | 1.5 | 0.65 |
| | 40 °C 2W | 0.04 | 98.6 | 1.4 | 0.70 | 97.6 | 1.7 | 0.71 |

| | | | | | | | | |
|---|---|---|---|---|---|---|---|---|
| Note: / represents not detected. * indicates that the data at 0h of R26 was used as a 0h control. | | | | | | | | |

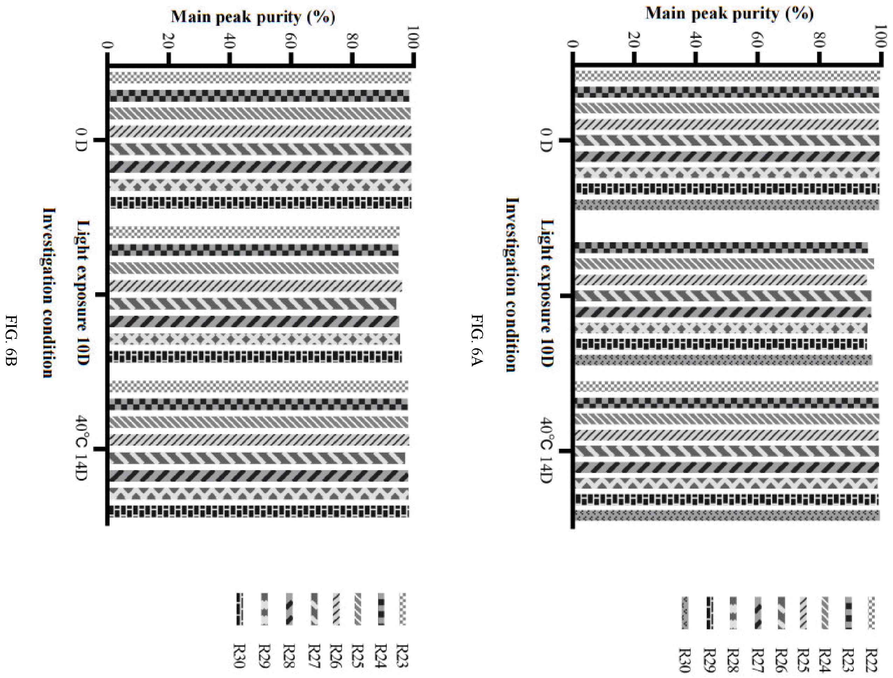

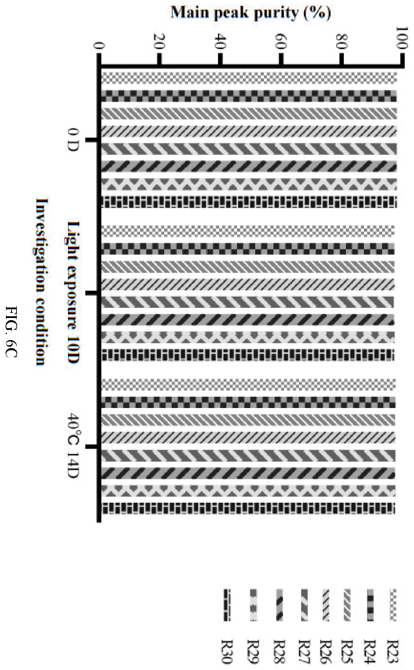

According to the data in Tables 10 and 11 and FIGs. 6A, 6B, and 6C, it can be seen that: after storage at 40 °C for 14 days, as compared with formula R23, the SEC-HPLC, nrCE-SDS, and rCE-SDS main peak purity of the self-buffering system formulas and other formulas containing the amino acid system had no significant difference; after storage under light exposure for 10 days, as compared with formula R23, the SEC-HPLC main peaks of formulas R24, R26, R27, and R30 were reduced by about 1.8%, 2.6%, 2.6%, and 2.3%, and the reductions were smaller than that of formula R23 (about 3.5% reduction in the SEC-HPLC main peak). The trends of changes in the SEC-HPLC main peaks of formulas R22, R25, R28, and R29 were substantially consistent with that of formula R23; the reductions in the nrCE-SDS and rCE-SDS main peaks of other groups were substantially consistent with that of formula R21. The results described above show that the formulation formulas containing a combination of arginine and proline or a combination of arginine and sucrose have excellent stability.

The embodiments of the present disclosure described above are merely exemplary, and any person skilled in the art can recognize or determine numerous equivalents of specific compounds, materials, and operations without the need for undue experimentation. All such equivalents shall fall within the scope of the present disclosure, and are encompassed by the claims.

## Claims

1. A pharmaceutical composition of an anti-PD-1 antibody, comprising
a) an anti-PD-1 antibody at about 80-200 mg/mL;
b) a surfactant, wherein the surfactant is selected from polysorbate 80, polysorbate 20, and poloxamer 188;
c) a stabilizer, wherein the stabilizer is a combination of arginine and proline, or a combination of arginine and sucrose;
d) an antioxidant, wherein the antioxidant is methionine;
e) a diffusing agent, wherein the diffusing agent is hyaluronidase,
wherein pH of the pharmaceutical composition is about 4.0-6.0, a content of the arginine is about 0.1-50 mg/mL, a content of the proline is about 0.1-50 mg/mL, and a content of the sucrose is about 0.1-50 mg/mL.

2. The pharmaceutical composition according to claim 1, wherein a weight ratio of the arginine to the proline or a weight ratio of the arginine to the sucrose is about 1:2 to 2:1, preferably about 1:2, 1:1, or 2:1.

3. The pharmaceutical composition according to claim 1, wherein the pharmaceutical composition comprises a buffer agent, wherein a content of the buffer agent is about 0.1-30 mM; preferably, the buffer agent is histidine-hydrochloric acid.

4. The pharmaceutical composition according to any one of claims 1-3, wherein a content of the surfactant is about 0.01-1 mg/mL.

5. The pharmaceutical composition according to any one of claims 1-3, wherein a content of the methionine is about 0.1-50 mM.

6. The pharmaceutical composition according to any one of claims 1-3, wherein nitrogen is introduced into the pharmaceutical composition.

7. The pharmaceutical composition according to any one of claims 1-3, wherein a content of the hyaluronidase is about 1-10000 IU/mL.

8. The pharmaceutical composition according to any one of the foregoing claims, wherein the anti-PD-1 antibody is pembrolizumab.

9. The pharmaceutical composition according to claim 8, wherein the heavy chain sequence of the pembrolizumab is shown in SEQ ID NO: 1, and the light chain sequence is shown in SEQ ID NO: 2.

10. A pharmaceutical composition of an anti-PD-1 antibody, comprising about 80-200 mg/mL pembrolizumab, about 0.1-30 mM histidine-hydrochloric acid, about 0.01-1 mg/mL polysorbate 80, about 0.1-50 mM methionine, about 0.1-50 mg/mL arginine, about 0.1-50 mg/mL proline, and about 1-10000 IU/mL hyaluronidase, wherein pH of the pharmaceutical composition is about 4.0-6.0, and optionally, nitrogen is introduced.

11. A pharmaceutical composition of an anti-PD-1 antibody, comprising about 160-180 mg/mL pembrolizumab, about 5-20 mM histidine-hydrochloric acid, about 0.1-0.6 mg/mL polysorbate 80, about 5-25 mM methionine, about 5-20 mg/mL arginine, about 5-20 mg/mL proline, and about 1-4000 IU/mL hyaluronidase, wherein pH of the pharmaceutical composition is about 5.0-6.0, and optionally, nitrogen is introduced.

12. The pharmaceutical composition according to claim 10, comprising:
(1) about 155 mg/mL pembrolizumab, about 1.5% arginine, about 1.5% proline, about 10 mM histidine-hydrochloric acid, about 0.4 mg/mL polysorbate 80, about 15 mM methionine, and about 2000 IU/mL hyaluronidase, wherein pH is about 5.5, and optionally, nitrogen is introduced;
(2) about 160 mg/mL pembrolizumab, about 1.5% arginine, about 1.5% proline, about 10 mM histidine-hydrochloric acid, about 0.4 mg/mL polysorbate 80, about 15 mM methionine, and about 2000 IU/mL hyaluronidase, wherein pH is about 5.5, and optionally, nitrogen is introduced;
(3) about 165 mg/mL pembrolizumab, about 1.5% arginine, about 1.5% proline, about 10 mM histidine-hydrochloric acid, about 0.4 mg/mL polysorbate 80, about 15 mM methionine, and about 2000 IU/mL hyaluronidase, wherein pH is about 5.5, and optionally, nitrogen is introduced;
(4) about 170 mg/mL pembrolizumab, about 2% arginine, about 1% proline, about 10 mM histidine-hydrochloric acid, about 0.4 mg/mL polysorbate 80, about 15 mM methionine, and about 2000 IU/mL hyaluronidase, wherein pH is about 5.5, and optionally, nitrogen is introduced;
(5) about 170 mg/mL pembrolizumab, about 1.5% arginine, about 1.5% proline, about 10 mM histidine-hydrochloric acid, about 0.4 mg/mL polysorbate 80, about 15 mM methionine, and about 2000 IU/mL hyaluronidase, wherein pH is about 5.5, and optionally, nitrogen is introduced;
(6) about 170 mg/mL pembrolizumab, about 1% arginine, about 2% proline, about 10 mM histidine-hydrochloric acid, about 0.4 mg/mL polysorbate 80, about 15 mM methionine, and about 2000 IU/mL hyaluronidase, wherein pH is about 5.5, and optionally, nitrogen is introduced;
(7) about 175 mg/mL pembrolizumab, about 1.5% arginine, about 1.5% proline, about 10 mM histidine-hydrochloric acid, about 0.4 mg/mL polysorbate 80, about 15 mM methionine, and about 2000 IU/mL hyaluronidase, wherein pH is about 5.5, and optionally, nitrogen is introduced;
(8) about 180 mg/mL pembrolizumab, about 1.5% arginine, about 1.5% proline, about 10 mM histidine-hydrochloric acid, about 0.4 mg/mL polysorbate 80, about 15 mM methionine, and about 2000 IU/mL hyaluronidase, wherein pH is about 5.5, and optionally, nitrogen is introduced;
(9) about 170 mg/mL pembrolizumab, about 1.5% arginine, about 1.5% proline, about 10 mM histidine-hydrochloric acid, about 0.5 mg/mL poloxamer 188, about 15 mM methionine, and about 2000 IU/mL hyaluronidase, wherein pH is about 5.5, and optionally, nitrogen is introduced;
(10) about 165 mg/mL pembrolizumab, about 2% arginine, about 2% sucrose, about 10 mM histidine-hydrochloric acid, about 0.4 mg/mL polysorbate 80, about 15 mM methionine, and about 2000 IU/mL hyaluronidase, wherein pH is about 5.0, and optionally, nitrogen is introduced;
(11) about 170 mg/mL pembrolizumab, about 2% arginine, about 2% sucrose, about 10 mM histidine-hydrochloric acid, about 0.4 mg/mL polysorbate 80, about 15 mM methionine, and about 2000 IU/mL hyaluronidase, wherein pH is about 5.5, and optionally, nitrogen is introduced;
(12) about 165 mg/mL pembrolizumab, about 2% arginine, about 2% sucrose, about 10 mM histidine-hydrochloric acid, about 0.4 mg/mL polysorbate 80, about 15 mM methionine, and about 2000 IU/mL hyaluronidase, wherein pH is about 5.5, and optionally, nitrogen is introduced.

13. A lyophilized formulation, wherein the lyophilized formulation is obtained by lyophilizing the pharmaceutical composition according to any one of the foregoing claims, or the lyophilized formulation, upon reconstitution, yields the pharmaceutical composition according to any one of the foregoing claims.

14. A method for treating a tumor, comprising administering to a subject the pharmaceutical composition or the lyophilized formulation according to any one of the foregoing claims.

15. The method according to claim 14, wherein the tumor is nasopharyngeal cancer, small cell lung cancer, non-small cell lung cancer, gastric cancer, esophageal cancer, pancreatic cancer, colorectal cancer, melanoma, kidney cancer, breast cancer, liver cancer, ovarian cancer, cervical cancer, bladder cancer, or head and neck tumor.
